# EUROPÄISCHE PATENTSCHRIFT

(11) **EP 0 451 765 B1**
(45) Veröffentlichungstag und Bekanntmachung des Hinweises auf die Patenterteilung: **24.08.1994**
(21) Anmeldenummer: 91105551.5
(22) Anmeldetag: 08.04.1991
(51) Int. Cl.: A61B 17/60

(54) **Knochenchirurgischer Halter**
Fixator for bone surgery
Fixateur pour chirurgie des os

(30) Priorität: 11.04.1990 DE 9004239 U
(43) Veröffentlichungstag der Anmeldung: 16.10.1991
(73) Patentinhaber: Waldemar Link (GmbH & Co.), D-22315 Hamburg (DE)
(72) Erfinder: Slot, G.H., Dr., NL-6522 JV Nijmegen (NL)
(74) Vertreter: Glawe, Delfs, Moll & Partner

(56) Entgegenhaltungen:
- WO-A-85/04096
- FR-A- 2 289 164
- FR-A- 2 624 720
- US-A- 4 573 454

## Beschreibung

Die Erfindung betrifft einen knochenchirurgischen Halter, der einen stangenförmigen, längeneinstellbaren Teil umfaßt.

Die Längenverstellung der stangenförmigen Teile von knochenchirurgischen Haltern nimmt man meist mit Hilfe von Gewindestangen vor, die aber den Nachteil haben, daß selbst eine geringfügige Längenverstellung eine vielfache Drehbewegung im Operationsfeld verlangt. Wünschenswert ist hingegen eine Einstellbarkeit, die mit weniger und einfacheren Bewegungen auskommt.

Bekannt ist es auch (FR-A-2,289,164), einen Teil mit einer Hülse und den anderen Teil mit einer in der Hülse gleitenden Stange zu versehen, wobei die Stange eine Reihe von sägezahnförmigen Rastvertiefungen trägt, mit denen Federzungen am Ende der Hülse zusammenwirken. Ausgehend von einer Längeneinstellung, die kürzer ist als die schließlich erforderliche, wird die Anordnung gestreckt, wobei die Federzungen der Hülse über die Rastelemente gleiten und schließlich in der passenden Rastvertiefung verbleiben und eine Verkürzung der Anordnung verhindern. Als wesentlicher Nachteil dieser Anordnung ist anzusehen, daß zum einen keine stufenlose Verstellung möglich ist und daß es zum anderen unmöglich ist, zu einer kürzeren Einstellung zurückzukehren, wenn man beim Ausprobieren der angemessenen Längeneinstellung feststellen muß, daß dies erforderlich ist.

Erfindungsgemäß ist vorgesehen, daß der stangenförmige, längeneinstellbare Teile eine dünnwandige Hülse aus einem plastisch verformbaren Metall und eine in der Hülse geführte Stange mit einer Mehrzahl von in Längsrichtung aufeinanderfolgenden Vertiefungen umfaßt. Für die Längeneinstellung genügt es, die beiden Teile teleskopisch in die gewünschte Einstellage zu bringen und diese durch Verformung der dünnen Hülse in eine oder mehrere Vertiefungen der Stange hinein zu fixieren.

Bei einer vorteilhaften Ausführungsform sind die Vertiefungen als Umfangsnuten ausgebildet. Mit anderen Worten, die Vertiefungen selbst sowie die zwischen ihnen liegenden Vorsprünge sind von Rotationsflächen begrenzt. Das hat zur Folge, daß die Stange und die Hülse auch noch nach der Längsfixierung gegeneinander verdrehbar sind, falls dies gewünscht wird.

Will man die Verdrehbarkeit sowohl vor der Fixierung als auch danach ausschließen, so stattet man die Hülse und die Stange mit übereinstimmenden, vom Kreisquerschnitt abweichenden Querschnittsformen aus, beispielsweise mit einem Oval- oder Polygonquerschnitt.

Wünscht man hingegen, daß die beiden Teile vor der Fixierung gegeneinander verdrehbar sind, danach aber nicht mehr, so gestaltet man die Vertiefungen in solcher Weise, daß sie Begrenzungen in Umfangsrichtung aufweisen. Beispielsweise ist jede einzelne Vertiefung als geradlinige, quer zur Stangenachse verlaufende Nut ausgebildet. Mehrere derartige Vertiefungen können auf einer oder auf mehreren, bestimmten Seiten der Stange in Längsrichtung hintereinander aufgereiht sein. Jede einzelne Vertiefung hat - bezogen auf die Umfangskontur der Stange - in der Mitte ihre größte Tiefe, während sie zu den Seiten hin sich der Umfangsfläche nähert und sie schließlich durchdringt, wodurch sich eine Kantenbegrenzung jeder Vertiefung bildet, die im Zusammenwirken mit dem in diese Vertiefung eindringenden, verformten Teil der Hülse einen Drehanschlag bildet.

Besonders geeignet ist der erfindungsgemäße Halter in Verbindung mit zwei Gewindestangen in der Form eines H-Rahmens zur Verwendung in der Wirbelsäulenchirurgie.

Dafür kann es auch vorteilhaft sein, wenn sowohl die Hülse als auch die Stange jeweils quer mit einer Steckzapfenkupplung versehen sind, die entweder als Zapfen oder als Hülse ausgebildet ist, um in entsprechender Weise mit dem Hülsen- oder Zapfenende einer daran anzusetzenden Stange zusammenzuwirken. Die Steckzapfenkupplung kann mit einer Verdrehsicherung versehen sein.

Die Erfindung wird im folgenden näher unter Bezugnahme auf die Zeichnung erläutert, die vorteilhafte Ausführungsbeispiele veranschaulicht. Es zeigen:
- Fig. 1: einen H-Rahmen
- Fig. 2 und 3: zwei Ansichten des einen H-Rahmenteils und
- Fig. 4 und 5: zwei Ansichten des anderen Teils,
- Fig. 6: die Verwendung des Halters gemäß Fig. 1 in der Wirbelsäulenchirurgie,
- Fig. 7: eine andere Ausführungsform des Halters und
- Fig. 8 - 13: dessen Bestandteile.

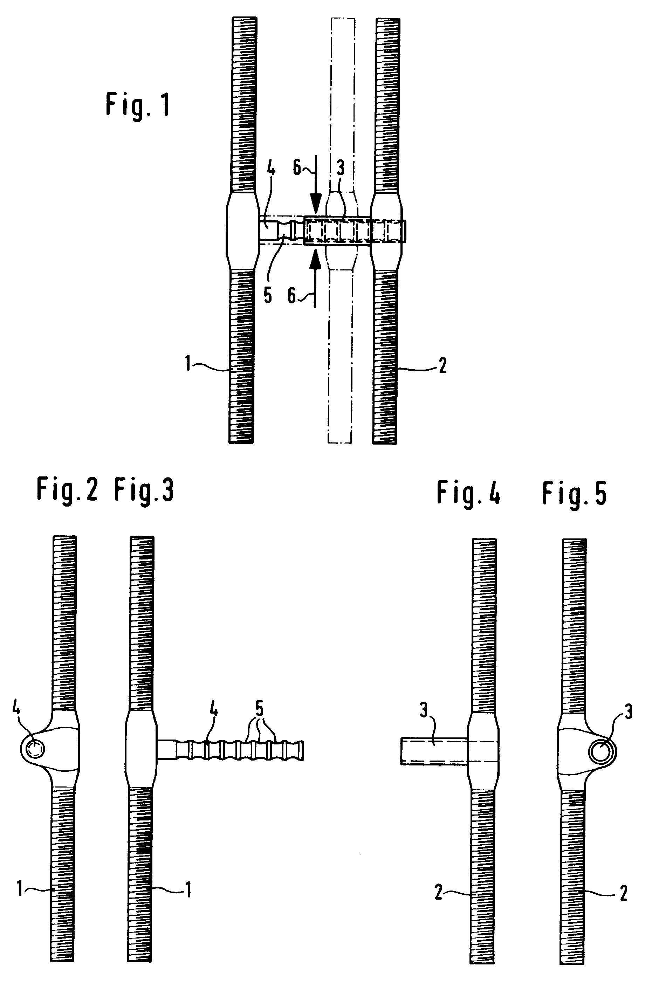

Der Halter gemäß Fig. 1 umfaßt zwei Gewindestangen 1, 2, die dadurch miteinander verbunden sind, daß die Gewindestange 2 in Querrichtung mit einer dünnwandigen Hülse 3 und die Gewindestange 1 in Querrichtung mit eine Stange 4 fest verbunden sind, wobei die letztere in der Hülse 3 passend geführt ist und Umfangsnuten 5 enthält. Die Stange 4 ist in der Hülse 3 zunächst frei verschieblich, so daß unterschiedliche Abstandseinstellungen, wie in Fig. 1 mit durchgezogenen und strichpunktierten Linien angedeutet, eingestellt werden können. Ist die gewünschte Einstellung gefunden, so kann mit einem geeigneten Kneifwerkzeug, dessen Backen durch die Pfeile 6 angedeutet sind, die Hülse 3 stellenweise in die Vertiefungen 5 der Stange 4 hinein verformt werden, wodurch die gegenseitige Einstellung dieser Teile in Längsrichtung gesichert wird.

Die Form der Bestandteile des Halters gemäß Fig. 1 geht aus den Fig. 2 bis 5 deutlicher hervor. Man erkennt darin, daß die Querverbindung der Gewindestangen 1 und 2 gegenüber der Ebene, in der diese sich befinden, ein wenig versetzt ist, damit die Gewindestangen 1, 2 in die Vertiefungen neben den Dornvorsprüngen der Wirbelsäule eingelegt werden können.

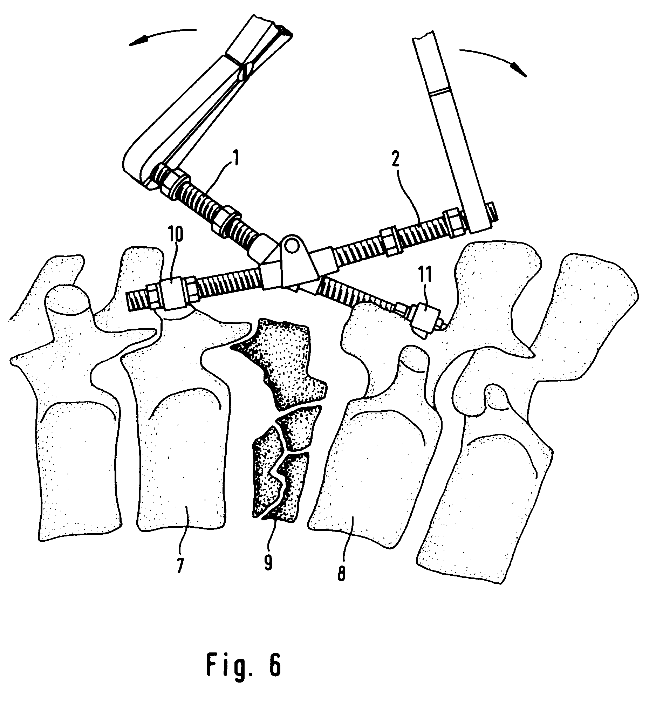

Ein besonderes Merkmal dieser Ausführungsform besteht darin, daß die Hülse 3 und die Stange 4 auch nach deren formschlüssiger Verbindung noch gegeneinander verdreht werden können. Dies gestattet die in Fig. 6 dargestellte Anwendung. Zur Distraktion der Wirbelkörper 7, 8 im Falle einer Fraktur des dazwischenliegenden Wirbelkörpers 9 werden die entgegengesetzten Enden der Gewindestangen 1, 2 mit Knochenhaken oder Knochenschrauben in bekannter Weise bei 10, 11 in solcher Weise verbunden, daß sie nicht parallel zueinander liegen, sondern einen stumpfen Winkel miteinander einschließen. Werden sie anschließend in Richtung der Pfeile mittels geeigneter Werkzeuge geschwenkt, was dank der Drehbarkeit der Stange 4 gegenüber der Hülse 3 möglich ist, so ergibt sich eine kniehebelartige Funktion, durch die die Wirbel 7 und 8 distrahiert werden. Wenn ihre Verbindung mit den Gewindestangen 1, 2 mittels starr mit den Gewindestangen verbundener Knochenschrauben erfolgt, ist damit gleichzeitig eine Aufrichtung dieser Wirbelkörper verbunden. Die freien Enden der Gewindestangen 1, 2 können anschließend gleichfalls an den Wirbelkörpern fixiert werden.

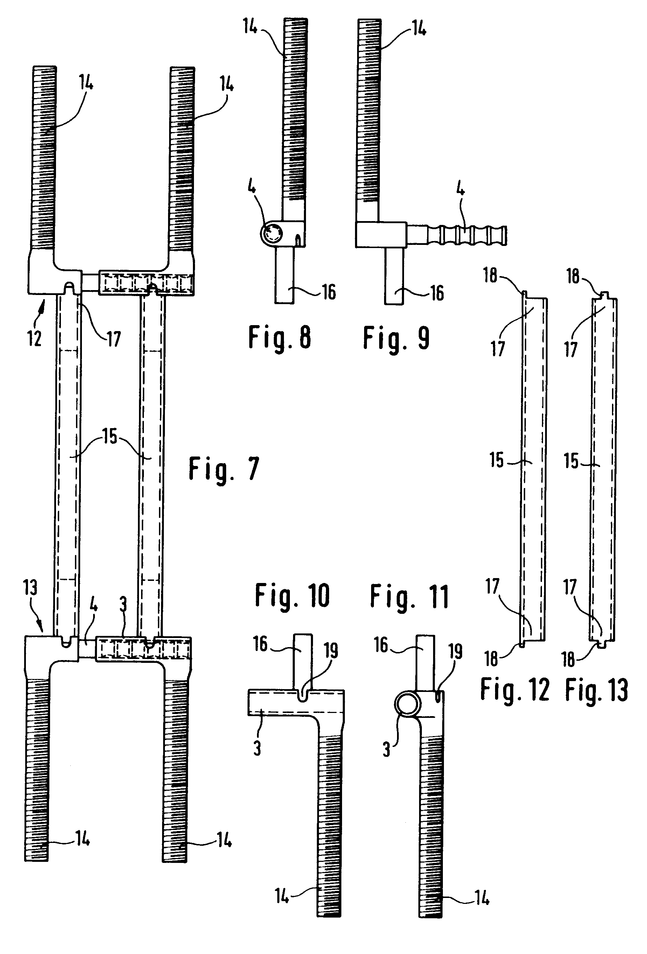

Die Ausführungsform gemäß Fig. 7 umfaßt zwei erfindungsgemäß ausgestattete Halter 12, 13 mit Gewindestangen 14, die miteinander durch ein Paar von Stangen oder Rohren 15 verbunden sind, die lösbar an die Halter 12, 13 ansteckbar sind. Zu diesem Zweck sind an die beiden Teile, aus denen die Halter 12, 13 jeweils bestehen, Steckzapfen 16 fest angebracht, die in die Hülsenenden 17 der Rohre oder Stangen 15 passen. Die Hülsenenden 17 und die Steckzapfen 16 werden zusammengehalten durch die Gegenkraft, die infolge der Knochendistraktion auf den Halter ausgeübt wird.

Damit sich die Halter 12, 13 nicht um die Längsachse der Anordnung gegeneinander verdrehen können, sind die Hülsenenden 17 mit einer Nase 18 und die Halter 12, 13 an entsprechender Stelle mit einem Ausschnitt 19 ausgerüstet, in den die Nase 18 eingreift.

Die Länge der stangenförmigen Verbindung zu den beiden Teilen, aus denen die Halter 12, 13 jeweils bestehen, ist in derselben Weise einstellbar, wie dies unter Bezugnahme auf Fig. 1 oben beschrieben wurde.

Die Ausführungen bestehen aus den für derartige Zwecke bewährten Werkstoffen. Zumindest die Hülse 3 besteht aus einem Werkstoff, der plastisch verformbar ist und die Fähigkeit hat, seine verformte Gestalt beizubehalten, im allgemeinen aus entsprechendem Metall.

## Patentansprüche

1. Knochenchirurgischer Halter, der einen stangenförmigen, längeneinstellbaren Teil aufweist, dadurch gekennzeichnet, daß der stangenförmige, längeneinstellbare Teil eine dünnwandige Hülse (3) aus einem plastisch verformbaren Metall und eine in der Hülse geführte Stange (4) mit einer Mehrzahl von in Längsrichtung aufeinanderfolgenden Vertiefungen (5) umfaßt.

2. Halter nach Anspruch 1, dadurch gekennzeichnet, daß die Vertiefungen (5) als Umfangsnuten ausgebildet sind.

3. Halter nach Anspruch 1, dadurch gekennzeichnet, daß die Vertiefungen (5) mit Begrenzungen in Umfangsrichtung versehen sind.

4. Halter nach einem der Ansprüche 1 bis 3, dadurch gekennzeichnet, daß er zwei Gewindestangen (1, 2; 14) zur Bildung eines H-Rahmens aufweist.

5. Halter nach einem der Ansprüche 1 bis 4, dadurch gekennzeichnet, daß mit der Hülse (3) und der Stange (4) jeweils quer eine Steckzapfenkupplung (16, 17) versehen ist.

6. Halter nach Anspruch 5, dadurch gekennzeichnet, daß die Steckzapfenkupplung mit einer Verdrehsicherung (18, 19) versehen ist.

## Claims

1. A retaining means for bone surgery, which has a rod-like, longitudinally adjustable member, characterised in that the rod-like, longitudinally adjustable member comprises a thin-walled sleeve (3) of a plastically deformable metal and a rod (4) which is guided in the sleeve and which has a plurality of indentations (5) following one another in longitudinal direction.

2. A retaining means according to Claim 1, characterised in that the indentations (5) are in the form of peripheral grooves.

3. A retaining means according to Claim 1, characterised in that the indentations (5) are provided with boundaries in peripheral direction.

4. A retaining means according to any one of Claims 1 to 3, characterised in that it has two threaded rods (1, 2; 14) for forming an H-frame.

5. A retaining means according to any one of Claims 1 to 4, characterised in that the sleeve (3) and the rod (4) are respectively provided transversely with a connector pin coupling (16, 17).

6. A retaining means according to Claim 5, characterised in that the connector pin coupling is provided with an antirotational feature (18, 19).

## Revendications

1. Fixateur pour chirurgie des os, qui présente une partie en forme de tige, réglable en longueur, caractérisé en ce que la partie en forme de tige, réglable en longueur comprend un manchon (3) à paroi fine constitué d'un métal plastiquement déformable et une tige (4) s'engageant dans le manchon ayant une multiplicité d'évidements (5) disposés consécutivement dans la direction longitudinale.

2. Fixateur selon la revendication 1, caractérisé en ce que les évidements (5) constituent des rainures circonférentielles.

3. Fixateur selon la revendication 1, caractérisé en ce que les évidements (5) présentent des délimitations circonférentielles.

4. Fixateur selon l'une des revendications 1 à 3, caractérisé en ce qu'il présente deux tiges filetées (1, 2; 14) formant un cadre en H.

5. Fixateur selon l'une des revendications 1 à 4, caractérisé en ce que le manchon (3) et la tige (4) présentent chacun un dispositif d'accouplement (16, 17) en pivot d'emboîtement disposé perpendiculairement.

6. Fixateur selon la revendication 5, caractérisé en ce que le dispositif d'accouplement en pivot d'emboîtement présente un dispositif d'arrêt (18, 19) s'opposant à la torsion.
